# EUROPEAN PATENT SPECIFICATION

(11) **EP 1 580 774 B1**
(45) Date of publication and mention of the grant of the patent: **09.04.2014**
(21) Application number: 03810664.7
(22) Date of filing: 07.11.2003
(51) Int. Cl.: H01G 9/035, H01G 9/02, H01G 9/04, H01G 9/10, H01M 6/18

(54) **ELECTROLYTIC CAPACITOR**
ELEKTROLYTKONDENSATOR
CONDENSATEUR ELECTROLYTIQUE

(30) Priority: 08.11.2002 JP 2002326019; 08.11.2002 JP 2002326028; 11.11.2002 JP 2002326718; 11.11.2002 JP 2002326720
(43) Date of publication of application: 28.09.2005
(73) Proprietor: NIPPON CHEMI-CON CORPORATION, Tokyo 141-0032 (JP); Mitsubishi Chemical Corporation, Chiyoda-ku Tokyo 100-8251 (JP)
(72) Inventor: Ozawa, Masashi, Tokyo 141-0032 (JP); Takeda, Masayuki, Tokyo 100-8251 (JP); Ue, Makoto, Tokyo 100-8251 (JP)
(74) Representative: Müller Schupfner & Partner
(86) International application number: PCT/JP2003/014217
(87) International publication number: WO 2004/042758

(56) References cited:
- EP-A- 0 684 620
- EP-A- 1 139 356
- EP-A- 1 394 824
- JP-A- 8 321 442
- JP-A- 2003 142 346
- US-B1- 6 459 565
- PATENT ABSTRACTS OF JAPAN vol. 1997, no. 04, 30 April 1997 (1997-04-30) -& JP 08 321442 A (MATSUSHITA ELECTRIC IND CO LTD; SANYO CHEM IND LTD), 3 December 1996 (1996-12-03)

## Description

### Field of the Invention

The present invention relates to an electrolytic capacitor, especially, the electrolytic capacitor having a low impedance characteristic and a high withstand voltage characteristic.

### Background of the Invention

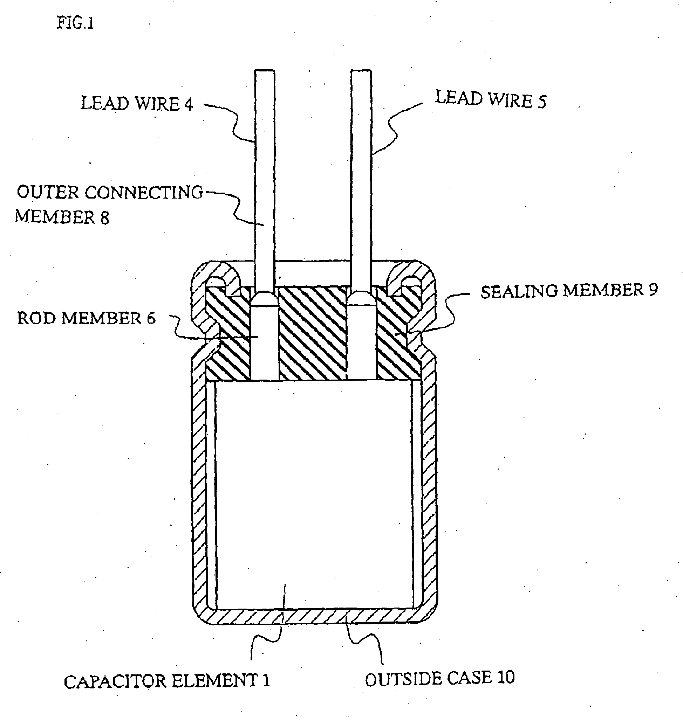

An electrolytic capacitor typically has such a structure shown in FIG.1. That is, an anode electrode foil 2 is made of a band-shaped high purity aluminum foil where the effective aluminum foil surface has been enlarged through etching process chemically or electrochemically, and an oxide film is formed on the surface, through a chemical process of treating the aluminum foil with a chemical solution such as ammonium borate aqueous solution and the like. A cathode electrode foil 3 is also made of an etched aluminum foil of high purity. Capacitor element 1 is formed by the anode electrode foil 2 and the cathode electrode foil 3, wound together with intervening separator 11 made of manila paper and the like. Next, the capacitor element 1, after impregnating with electrolyte solution for driving the electrolytic capacitors, is housed into a bottomed outer case 10 made of aluminum and the like. The outer case 10 is equipped at the opening with a sealing member 9 made of an elastic rubber, and is sealed by drawing.

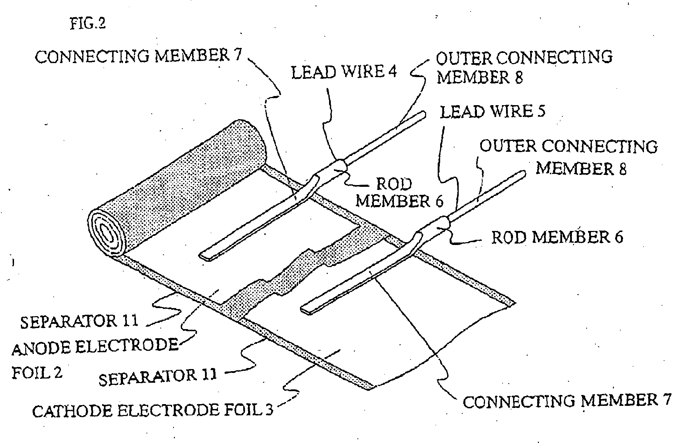

The anode electrode foil 2 and the cathode electrode foil 3 are each connected to lead wires 4 and 5, employed as electrode leading means to lead the electrodes, by means of stitching, ultrasonic welding, and the like, as shown in FIG.2. Each of the lead wires 4 and 5 employed as electrode leading means is comprised of a rod member 6 made of aluminum, and a connecting member 7 that comes into contact with each of the electrode foils 2 and 3, and further an outside connecting member 8 made of solderable metal which has been fixed at the tip of the rod member 6.

Herewith, as electrolyte solution for driving the electrolytic capacitor having high conductivity, and to be impregnated to the capacitor element, wherein γ-butyrolactone is employed as the main solvent composed of quaternized cyclic amidin compounds (imidazolinium cation and imidazolium cation) as the cationic component and acid conjugated bases as the anionic component are dissolved therein as the solute (refer to Unexamined Published Japanese Patent Application No. H08-321449 and No. H08-321441)

However, due to the remarkable development of digital information devices in recent years, the high-speed driving frequency of micro-processor which is a heart of these electronic information devices is in progress. Accompanied by the increase in the power consumption of electronic components in the peripheral circuits, the ripple current is increased abnormally, and there is a strong demand for the electrolytic capacitors used in these circuits to have a low impedance characteristic.

Moreover, in the field of vehicles, with the recent tendency toward improved automobile functions, a low impedance characteristic is in high demand. By the way, the driving voltage of the vehicle circuit of 14V has been progressed to 42V accompanied by the increase in the power consumption. To comply with such a driving voltage, the electrolytic capacitor requires the withstand voltage characteristic of 28V and 84V and more. Furthermore, the electrolytic capacitors must withstand high temperature in this field, and a high temperature life characteristic is in demand.

However, the electrolytic capacitor cannot cope with the low impedance characteristic as such. Moreover, although the withstand voltage of 28V is capable, the limit is 30V, and it cannot respond to the requirement of the high withstand voltage of 84V and more. Moreover, these electrolytic capacitors suffer from a problem that the moisture resistant characteristic is low despite of the fact that the moisture resistance of these electrolytic capacitors are in demand similar to the semiconductors.

EP 1 394 824 A1 a document according to Article 54 (3) EPC, hence relevant for novelty, discloses an electrolytic capacitor according to the pre-characterizing portion of claim 1.

Henceforth, the present invention aims to provide an electrolytic capacitor having a low impedance characteristic and a high withstand voltage characteristic of 100V class, and an excellent high temperature life characteristic and an excellent moisture resistant characteristic.

### Disclosure of Invention

This aim is achieved with an electrolytic capacitor according to claim 1. Advantageous embodiments of the invention are subject-matter of the dependent claims.

### Brief Description of Drawings

FIG.1 is an inner cross-sectional view showing a structure of electrolytic capacitor; and
FIG.2 is a decompositional oblique view showing a structure of electrolytic capacitor.

### Best Mode to Carrying Out the Invention

Aluminum electrolytic capacitor has such a structure same as the conventional structure, as shown in FIGS. 1 and 2. Capacitor element 1 is formed by an anode electrode foil 2 and a cathode electrode foil 3, wound together with intervening separator 11. Moreover, as shown in FIG.2, lead wires 4 and 5, employed as the electrode leading means, are connected to the anode electrode foil 2 and the cathode electrode foil 3, respectively. The lead wires 4 and 5 are comprised of connecting members 7 that come into contact with both electrode foils; rod members 6 connected to the connecting members 7; and an outer connecting member 8 weld to the rod member 6. Further, each foil and lead wire is mechanically connected by means of stitching, ultrasonic welding, and the like.

The anode electrode foil 2 used is one obtained in such a manner that an aluminum foil of a purity of 99% is subjected to chemical or electrochemical etching in an acidic solution to enhance the surface area thereof and then subjected to chemical treatment in an ammonium borate or ammonium adipate aqueous solution, so as to form an anode oxide film layer on the surface thereof.

The capacitor element 1 impregnating with the electrolyte solution is housed in an aluminum cylindrical outer case 10 with a bottom, and a sealing member 9, having a perforation hole for guiding the lead wires 4 and 5, is inserted into an open end of the outer case 10, and further, the open end of the outer case 10 is sealed by drawing to seal the aluminum electrolytic capacitor.

Then, a separator of the present invention is made of heat-resistant synthetic resin. Examples of the separator include fabric, nonwoven fabric, paper, and porous film. In other words, the fabric, nonwoven fabric or paper made by using the high-molecular fibers such as polyester, polyamide, vinylon, rayon, aramid, poly ethylene terephthalate, polyethylene naphtahalate, poly phenylene sulfide, aromatic polyester, polyimide, polyamido-imido, polyetherimide, polytetrafluoroethylene, polyaminobismaleimide, poly(ethylene-tetraethylene), poly(vinylidene fluoride), and the like, or using the high porous film made by using these high molecules. Examples of resins used as binders include epoxy resin, phenol resin, polyurethene resin, and melamine resin. Due to the low tensile strength and low heat resistance nature of polypropylene, polyethylene and the like, winding of the capacitor element is going to be difficult using these and thus not preferable

The electrolyte solution of the electrolytic capacitor used in the present invention contains an aluminum tetrafluoride salt.

As the aluminum tetrafluoride salt constituting the aluminum tetrafluoride as anion component, examples of this salt include an ammonium salt, an amine salt, a quaternary ammonium salt, or a quaternary cyclic amidinium ion as cation component, can be used. Examples of an amine constituting the amine salt include a primary amine (such as methylamine, ethylamine, propylamine, butylamine, ethylenediamine, monoethanolamine, and the like); secondary amine (such as dimethylamine, diethylamine, dipropylamine, ethy-methylamine, diphenylamine, diethanolamine and the like); and tertiary amine (such as trimethylamine, triethylamine, tributylamine, triethanolamine, and the like). Examples of a quaternary ammonium constituting the quaternary ammonium salt include a tetraalkylammonium (such as tetramethylammonium, tetraethylammonium, tetrapropylammonium, tetrabutylammonium, methyltriethylammonium, di-methyldiethylammonium and the like) and a pyridinium (such as 1-methylpyridinium, 1-ethylpyridinium, 1,3-diethylpyridinium and the like).

Furthermore, as for salt containing the quaternized cyclic amidinium ion as a cationic component, the quaternized cyclic amidinium ion is a cation formed by quaternized a cyclic compound having an N,N,N'-substituted amidine group, and the following compounds are exemplified as the cyclic compound having an N,N,N'-substituted amidine group. They are an imidazole monocyclic compound (for example, an imidazole homologue, such as 1-methylimidazole, 1-phenylimidazole, 1,2-dimethyl-imidazole, 1-ethyl-2-methylimidazole, 2-ethyl-1-methylimidazole, 1,2-diethylimidazole, 1,2,4-trimethylimidazole and the like, an oxyalkyl derivative, such as 1-methyl-2-oxymethylimidazole, 1-methyl-2-oxyethyl-imidazole, and the like, a nitro derivative such as 1-methyl-4(5)-nitroimidazole, and the like, and an amino derivative such as 1,2-dimethyl-5(4)-aminoimidazole, and the like), a benzoimidazole compound (such as 1-methylbenzoimidazole, 1-methyl-2-benzylbenzoimidazole, 1-methyl-5(6)-nitrobenzo-imidazole and the like), a compound having a 2-imidazoline ring (such as 1-methylimidazoline, 1,2-dimethylimidazoline, 1,2,4-trimethylimidazoline, 1-methyl-2-phenylimidazoline, 1-ethyl-2-methylimidazoline, 1,4-dimethyl-2-ethyl-imidazoline, 1-methyl-2-ethoxymethylimidazoline, and the like), a compound having a tetrahydropyrimidine ring (such as 1-methyl-1,4,5,6-tetrahydropyrimidine, 1,2-dimethyl-1,4,5,6-tetrahydropyrimidine, 1,8-diazabicyclo[5,4,0] undecen-7,1,5-diazabicyclo[4,3,0]-nonene-5, and the like), and the like.

The solvent in use for electrolyte solution according to the present invention comprises a polar protic solvent, a polar aprotic solvent, and their mixture thereof. Examples of the polar protic solvent include monohydric alcohols (such as ethanol, propanol, butanol, pentanol, hexanol, cyclo-butanol, cyclo-pentanol, cyclo-hexanol, benzyl alcohol, and the like); and polyhydric alcohol and oxy alcohol compounds (such as ethylene glycol, propylene glycol, glycerine, methyl cellosolve, ethyle cellosolve, methoxy propylene glycol, dimethoxy propanol, and the like). Moreover, representative examples of the aprotic polar solvent include amide series (such as N-methylformamide, N,N-dimethylformamide, N-ethylformamide, N,N-diethylformamide, N-methyl acetamide, N,N-dimethyl acetamide, N-ethyl acetamide, N,N-diethyl acetamide, hexamethylphosphoric amide, and the like); lactone compounds (such as γ-butyrolactone, δ -valerolactone, γ-valerolactone, and the like); sulfolane series (such as sulfolane, 3-methyl sulfolane, 2,4-dimethyl sulfolane, and the like); cyclic amide compounds (such as N-methyl-2-pyrrolidone, and the like); carbonates (such as ethylene carbonate, propylene carbonate, isobutylene carbonate, and the like); nitrile compound (such as acetonitrile, and the like); sulfoxide compound (such as dimethyl sulfoxide, and the like); 2-imidazolidinone solvents [for example, 1,3-dialkyl-2-imidazoridinone (such as 1,3-dimethyl-2-imidazoridinone, 1,3-diethyl-2-imidazoridinone, 1,3-di(n-propyl)-2-imidazoridinone, and the like); and 1,3,4-trialkyl-2-imidazoridinone (such as 1,3,4-trimethyl-2-imidazoridinone, and the like)], and the like. Among them, γ-butyrolactone is preferably used because the impedance characteristic improves. Sulfolane, 3-methyl sulfolane, and 2,4-dimethyl sulfolane are preferably used because the high temperature characteristic improves. Ethylene glycol is preferably used because the withstand voltage characteristic improves.

Accordingly, the electrolyte solution containing aluminum tetrafluoride salt is used in the electrolytic capacitor of the present invention. The electrolytic capacitor of the present invention has a low impedance characteristic, and a high temperature withstand voltage characteristic. Because the separator in use is made of heat resistant synthetic resin, moisture from the separator is less likely to be mixed into the electrolyte solution, so that the electrolytic capacitor has the excellent high temperature life characteristic. That is to say, in case of using a separator from the conventional manila paper and the like, the moisture is generated from the separator, and the reactivity of the electrolyte solution used in the present invention with the electrode foil gets large to influence the life characteristic. However, in the present invention, such moisture generation is controlled to obtain an excellent high temperature life characteristic. Furthermore, the moisture resistance characteristic is excellent.

According to a first electrolytic capacitor of the present invention described above has the low impedance characteristic and the high withstand voltage characteristic of 100V class, wherein the electrolytic capacitor provides the excellent high temperature life characteristic and the excellent moisture resistance characteristic.

Subsequently, the second electrolytic capacitor of the present invention will be explained. The electrolytic capacitor of the present invention comprises a capacitor element fabricated by winding an anode foil and a cathode foil via a separator is impregnated with electrolyte solution, an outer case for housing the capacitor element, a sealing member for sealing an open part of the outer case, wherein the electrolyte solution in use contains an aluminum tetrafluoride salt, and wherein the separator in use is a mixed paper containing glass fiber.

The electrolytic capacitor has the same structure as the first electrolytic capacitor. As separator, a mixed paper containing glass fiber is used in the present invention. Examples of the mixed fibers include pulp fiber used in papers such as manila paper, craft paper and the like; and the synthetic fibers such as polyester fiber, polyethylene fiber, polypropylene fiber, polytetrafluoroethylene fiber, polyamido fiber, and the like. When a separator made only from glass fiber is used, a thickness of the separator increases, and the impedance of electrolytic capacitor gets large. The effectiveness of the electrolytic capacitor of the present invention is not obtainable by using this.

Accordingly, the electrolyte solution containing aluminum tetrafluoride salt is used in the electrolytic capacitor of the present invention. The electrolytic capacitor of the present invention has a low impedance characteristic and a high temperature withstand voltage characteristic. Because the separator in use is made of mixed paper containing glass fiber, the moisture from the separator is less likely to be mixed into the electrolyte solution, so that the electrolytic capacitor has the excellent high temperature life characteristic. That is to say, in case of using a separator made from the conventional manila paper and the like, the moisture is generated from the separator, and the reactivity of the electrolyte solution used in the present invention with the electrode foil gets large to influence the life characteristic. However, such the present invention, the moisture generation is controlled to obtain an excellent high temperature life characteristic. Furthermore, the moisture resistance characteristic is excellent.

The second electrolytic capacitor of the present invention described above has the low impedance characteristic, the high withstand voltage characteristic of 100V class, and the excellent moisture resistance characteristic.

Further, the first and second electrolytic capacitors of the present invention will be described. The electrode foil subjected to phosphate treatment is used as the electrode foils. The present invention is still effective by using the electrode foil subjected to phosphate treatment as one of the cathode electrode foil and the anode electrode foil. Dcterioration of both foils is prevented if this is applied to both foils so normally both foils are subjected to phosphate treatment. Normally, the aluminum foil of high purity is subjected to chemical or electrochemical etching to obtain the etching foil, however, as the electrode foil of the present invention, the etching foil obtained by performing the phosphate aqueous solution impregnation process before, during, or after the etching process is used as the cathode electrode foil. Further, as the anode electrode foil, the etching foil, the etching foil untreated with phosphate is subjected to phosphate synthesis, or the electrode foil that performed the phosphate impregnation process before, during, or after the chemical treatment is used.

Furthermore, the effect of the present invention improves by adding the phosphorous compounds to the electrolyte solution of the electrolytic capacitor described above. Examples of phosphorus compounds and salts thereof include orthophosphoric acid, phosphonous acid, hypophosphorus acid and their salts. As the salts of the phosphorus compounds, an ammonium salt, an aluminum salt, a sodium salt, a calcium salt, and a potassium salt can be used. Moreover, examples of phosphorous compound include ethyl phosphate, diethyl phosphate, butyl phosphate, dibutyl phosphate and the like; and phosphonate such as 1-hydroxyethylidene-1,1-diphosphonic acid, aminotrimethylene phosphonic acid, phenyl phosphonic acid, and the like. Moreover, examples of phosphinate include methyl phosphinate, butyl phosphinate, and the like.

Furthermore, examples of condensed phosphates include straight-chain condensed phosphates such as pyrophosphoric acid, tripolyphosphoric acid, tetrapolyphosphoric acid, and the like; cyclic condensed phosphates such as metaphosphate, hexametaphosphate, and the like, or the combination of the chain condensed phosphate and cyclic condensed phosphate. Further, as salts of these condensates, an ammonium salt, an aluminum salt, a sodium salt, a calcium salt, a potassium salt, and the like can be used.

The addition amount is ranging from 0.05 to 3% by weight, and preferably is ranging from 0.1 to 2% by weight.

The electrolytic capacitor of the present invention described above has the low impedance characteristic and the high withstand voltage of 100V class, and the excellent high temperature life characteristic. In other words, in case of performing the high temperature life test by using the aluminum tetrafluoride salt, the reactivity of the electrolyte solution with the electrode foil gets large due to the moisture inside the electrolyte solution, and the characteristics are affected. However, since the electrolytic capacitor of the present invention utilizes the electrode foil subjected to phosphate treatment, the reaction of the electrode foil with the electrolyte solution is controlled, whereby the high temperature life characteristic is stabilized.

Furthermore, similarly, in the present invention, a partial cross-linking peroxide butyl rubber that added peroxide as cross-linking agent to a butyl rubber polymer comprised of isobutylene, isoprene, and divinylbenzene copolymer is used as the sealing member. Examples of vulcanizing agents used in the vulcanization of peroxides include ketone peroxides, peroxy ketals, hydro-peroxides, dialkyl peroxides, diacyl peroxides, peroxy dicarbonates, peroxy esters, and the like. Specific examples are 1,1-bis-t-butylperoxy-3,3,5-trimethylcyclohexane, n-butyl-4,4-bis-t-butylperoxy-valerate, dicumyl peroxide, t-butyl-peroxy-benzoate, di-t-butyl-peroxide, benzoyl peroxide, 1,3-bis (t-butyl peroxy-isopropyl) benzene, 2,5-dimethyl-2,5-di-t-butylperoxyl-hexene-3, t-butyl peroxy cumene, α, α' bis (t-butylperoxy) diisopropylbenzene, and the like.

According to the electrolytic capacitor of the present invention, a partial cross-linking peroxide butyl rubber that added peroxide as cross-linking agent to a butyl rubber polymer comprised of isobutylene, isoprene, and divinylbenzene copolymer is used as the sealing member. The electrolyte solution containing the aluminum tetrafluoride salt is used. The electrolytic capacitor of the present invention has a low impedance characteristic, and a high withstand voltage characteristic of 100V class. The high temperature life characteristic is improved further by the excellent high temperature characteristics of the electrolyte solution and the sealing member of the present invention.

Moreover, the quaternary cyclic amidinium compound tends to cause leakage due to the reaction with the hydroxyl ion generated in the vicinity of the cathode leading means, however, the electrolyte solution used in the present invention seemingly has a less reactivity with the hydroxyl ion, and owing to the excellent sealability between the perforation hole of the sealing member and the lead wire, the leakage characteristic is further improved by these synergistic effects.

### (Embodiments)

Subsequently, the first invention will be explained by using the embodiments. The electrolytic capacitor of the present invention has the same structure as that of the conventional ones. The present invention is explained by referring to FIGS. 1 and 2. A capacitor element 1 is formed by winding an anode electrode foil 2 and a cathode electrode foil 3 via a separator 11. As FIG. 2 shows, the anode electrode foil 2 and the cathode electrode foil 3 are connected respectively to a lead wire 4 for leading the anode electrode and an another lead wire 5 for leading the cathode electrode.

These lead wires 4 and 5 are composed of connecting members 7 being in contact with the electrode foils, the rod members 6 having been molded integrally with the connecting members 7, and outer connecting members 8 having been fixed at the tip of the rod members 6. The connecting member 7 and the rod member 6 are made from aluminum of 99% purity while the outer connecting member 8 is made of a copper-plated steel wire (hereinafter CP wire). On the surfaces of the rod members 6 of the lead wires 4 and 5 at least, anode oxide films made of aluminum oxide are formed by a chemical treatment with ammonium phosphate aqueous solution. These lead wires 4 and 5 are connected respectively to the electrode foils 2 and 3 at the connecting members 7 by means of stitching, ultrasonic welding, and the like.

The anode electrode foil 2 is made of an aluminum foil of 99.9% purity in an acidic solution thereby enlarging the surface area thereof through the chemical or electrochemical etching process, and then subjecting the aluminum foil to a chemical treatment in an ammonium adipate aqueous solution, to thereby form an anode oxidation film on the surface thereof.

The capacitor element 1, which impregnates the electrolyte solution, is then housed into a bottomed outer case 10 made of aluminum. The outer case 10 is provided at the opening with a sealing member 9 and then sealed by drawing. The sealing member 9 is made of, for example, an elastic rubber such as butyl rubber, and the like, and has perforation holes through which the lead wires 4 and 5 are to be passed.

Further, as separator in use includes a separator composed of poly ethylene terephthalate (PET), and a conventionally used separator made of manila paper.

Moreover, the electrolyte solution A containing 75% by weight of γ -butyrolactone as solvent and 25% by weight of 1-ethyl-2,3-dimethylimidazolinium aluminum tetrafluoride salt as solute is used. The electrolyte solution B containing 80% by weight of γ -butyrolactone as solvent and 20% by weight of 1-ethyl-2,3-dimethylimidazolinium aluminum tetrafluoride salt as solute is used. Further, the electrolyte solution C containing 75% by weight of γ -butyrolactone as solvent and 1-ethyl-2,3-dimethylimidazolinium hydrogen phthalate salt as solute is used as the electrolyte solution containing conventionally used electrolyte.

The rated voltages of the electrolytic capacitors using the electrolyte solutions A and C are 16V, and that of using the electrolyte solution B is 100V. The characteristics of the electrolytic capacitors are evaluated. The test condition is 125°C at 2,000 hours in the loaded state, and 105ºC at 2,000 hours in the unloaded state. The results are shown in (Table 1-1) to (Table 1-4).

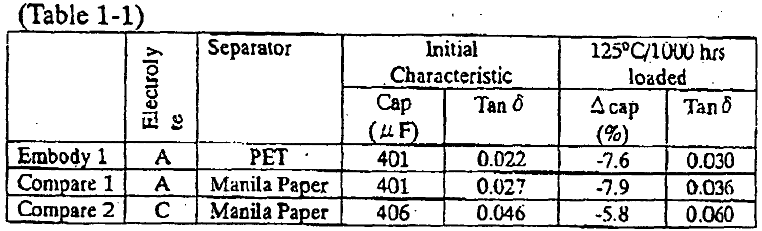

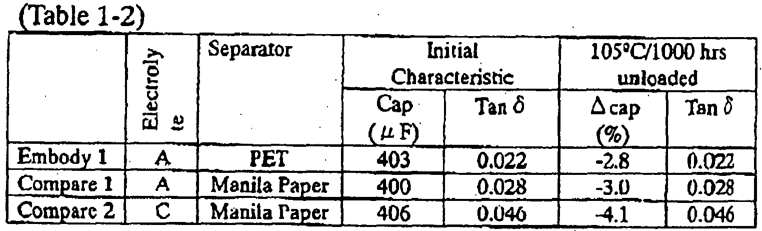

**(Table 1-3)**

| | Elect rolyte | Separator | Initial Characteristic | | 125°C/1000 hrs loaded | |
|---|---|---|---|---|---|---|
| | | | Cap (*µ*F) | Tan δ | Δ Cap (%) | Tan δ |
| Embody 2 | B | PET | 22.7 | 0.010 | -2.1 | 0.015 |
| Compare 3 | B | Manila Paper | 22.9 | 0.011 | -2.3 | 0.018 |

**(Table 1-4)**

| | Electrol yte | Separator | Initial Characteristic | | 125°C/1000 hrs unloaded | |
|---|---|---|---|---|---|---|
| | | | Cap (*µ*F) | Tan δ | Δ cap (%) | Tan δ |
| Embody 2 | B | PET | 22.8 | 0.010 | -1.6 | 0.010 |
| Embody 3 | B | Manila Paper | 22.9 | 0.011 | -2.0 | 0.012 |

As (Table 1-1) and (Table 1-2) clearly show, the electrolytic capacitor of the first embodiment has the excellent high temperature life characteristics, a low dielectric loss coefficient (tan δ), and a less change in the dielectric loss coefficient (tan δ) at 125ºC, compared with the electrolytic capacitor of the comparative examples 1 and 2. Furthermore, (Table 1-3) and (Table 1-4) clearly show the excellent life characteristics and initial characteristics of the rated voltage 100V, to implement the 100V class electrolytic capacitor having a low impedance characteristic not found in the conventional ones.

Subsequently, the moisture resistance characteristics of the electrolytic capacitors of the first embodiment and the first comparative example are evaluated. The test conditions are 85ºC, 85%RH, at 1.000 hours in the unloaded state. The results are shown in (Table 1-5).

**(Table 1-5)**

| | Electrol yte | Separator | Initial Characteristic | | 85°C/85%RH 1000 hrs unloaded | |
|---|---|---|---|---|---|---|
| | | | Cap (*µ*F) | Tan δ | Δ cap (%) | Tan δ |
| Embody 1 | A | PET | 402 | 0.022 | -2.0 | 0.024 |
| Compare 1 | A | Manila Paper | 401 | 0.028 | -2.3 | 0.030 |

As (Table 1-5) clearly shows, the electrolytic capacitor of the present invention have the excellent characteristics in the change in electrostatic capacity and the dielectric loss coefficient. The moisture resistance characteristic of the electrolytic capacitor of the present invention has improved.

Subsequently, the second electrolytic capacitor of the present invention will be described. This electrolytic capacitor has the same structure as that of the first electrolytic capacitor, and the contents of characteristic evaluation which are also the same. As separator in use include a separator composed of mixed paper containing glass fiber and a conventionally used separator made of manila paper. The results are shown in (Table 2-1) and (Table 2-4).

**(Table 2-1)**

| | Electrol yte | Separator | Initial Characteristic | | 125ºC/1000 hrs loaded | |
|---|---|---|---|---|---|---|
| | | | Cap (*µ*F) | Tan δ | Δ cap (%) | Tan δ |
| Embody 3 | A | Glass fiber | 403 | 0.022 | -7.5 | 0.030 |
| Compare 4 | A | Manila Paper | 400 | 0.027 | -7.8 | 0.036 |
| Compare 5 | C | Manila Paper | 405 | 0.046 | -5.7 | 0.060 |

**(Table 2-2)**

| | Electrol yte | Separator | Initial Characteristic | | 105°C/1000 hrs unloaded | |
|---|---|---|---|---|---|---|
| | | | Cap (*µ*F) | Tan δ | Δcap (%) | Tan δ |
| Embody 3 | A | Glass fiber | 404 | 0.022 | -2.9 | 0.022 |
| Compare 4 | A | Manila Paper | 401 | 0.028 | -3.2 | 0.028 |
| Compare 5 | C | Manila Paper | 405 | 0.046 | -4.1 | 0.046 |

**(Table 2-3)**

| | Electrol yte | Separator | Initial Characteristic | | 125°C/1000 hrs loaded | |
|---|---|---|---|---|---|---|
| | | | Cap (*µ*F) | Tan δ | Δ cap (%) | Tan δ |
| Embody 4 | B | Glass fiber | 22.7 | 0.011 | -2.2 | 0.015 |
| Compare 6 | B | Manila Paper | 22.8 | 0.011 | -2.5 | 0.018 |

**(Table 2-4)**

| | Electrol yte | Separator | Initial Characteristic | | 105°C/1000 hrs unloaded | |
|---|---|---|---|---|---|---|
| | | | Cap (*µ*F) | Tan δ | Δ cap (%) | Tan δ |
| Embody 4 | B | Glass fiber | 22.7 | 0.010 | -1.7 | 0.010 |
| Compare 6 | B | Manila Paper | 22.9 | 0.011 | -2.2 | 0.012 |

As (Table 2-1) and (Table 2-2) clearly show, the electrolytic capacitor of this embodiment has excellent high temperature life characteristics, a less change in dielectric loss coefficient (tan δ) of 125°C, and a low dielectric loss coefficient (tan δ), compared with the electrolytic capacitor of the comparative example. Furthermore, (Table 2-3) and (Table 2-4) clearly show the excellent life characteristics and initial characteristics of the rated voltage 100V, to implement the 100V class electrolytic capacitor having the low impedance characteristic not found in the conventional ones.

Subsequently, the moisture resistance characteristics of the electrolytic capacitors of the third embodiment and the fourth comparative example are evaluated. The test conditions are 85°C, 85%RH, at 4,000 hours in the unloaded state. The results are shown in (Table 2-5).

**(Table 2-5)**

| | Electrol yte | Separator | Initial Characteristic | | 85ºC/85%RH/ 1,000 hrs | |
|---|---|---|---|---|---|---|
| | | | Cap (*µ*F) | Tan δ | Δ cap (%) | Tan δ |
| Embody 3 | A | Glass fiber | 403 | 0.022 | -2.1 | 0.024 |
| Embody 4 | A | Manila Paper | 402 | 0.028 | -2.4 | 0.030 |

As (Table 2-5) clearly shows, the electrolytic capacitor of the present invention have the excellent characteristics in the change in electrostatic capacity and the dielectric loss coefficient. The moisture resistance characteristic of the electrolytic capacitor of the present invention has improved.

As for first second third electrolytic capacitors, in case of using an electrode foil subjected to phosphate treatment as the anode electrode foil and the cathode electrode foil, the high temperature life characteristic improved further. The high temperature life characteristic also improves by adding phosphorous compound to the electrolyte solution. Moreover, in case of using, as the sealing member, a partial cross-linking peroxide butyl rubber that added peroxide as cross-linking agent to a butyl rubber polymer comprised of isobutylene, isoprene, and divinylbenzene copolymer. Namely, the present invention achieves an extremely remarkable effect of preventing liquid leakage.

### INDUSTRIAL APPLICABILITY

According to the present invention, the electrolyte solution containing the aluminum tetrafluoride salt is used. As separator in use include a separator composed of heat resistant synthetic resin or a mixed paper containing glass fiber. Thus the electrolytic capacitor having the low impedance characteristic and the high voltage characteristic and the excellent high temperature life characteristic and the moisture resistance characteristic is supplied.

## Claims

1. An electrolytic capacitor having a capacitor element (1) fabricated by winding an anode foil (2), a cathode foil (3) and a separator (11) and impregnating it with an electrolyte solution, an outer case (10) for housing the capacitor element (1), and a sealing member (9) for sealing an open part of the outer case (10), wherein an electrolyte solution containing aluminum tetrafluoride salt is used as said electrolyte solution, and wherein the separator is made of a heat resistant synthetic resin,
**characterized in that** the heat resistant synthetic resin is polyester, polyamide, vinylon, rayon, aramid, poly ethylene terephthalate, polyethylene naphtahalate, poly phenylene sulfide, aromatic polyester, polyimide, polyamido-imido, polyetherimide, polytetrafluoroethylene, polyaminobismaleimide, poly(ethylene-tetraethylene), poly(vinylidene fluoride), or any combination thereof.

2. The electrolytic capacitor according to claim 1, wherein an electrode foil subjected to a phosphate treatment is used as the anode electrode foil (2) or the cathode electrode foil (3).

3. The electrolytic capacitor according to claim 1, wherein a partial cross-linking peroxide butyl rubber that adds peroxide as cross-linking agent to a butyl rubber polymer comprising a copolymer of isobutylene, isoprene, and divinylbenzene is used as the sealing member (9).

## Patentansprüche

1. Elektrolytkondensator mit einem Kondensatorelement (1), das durch Wickeln einer Anodenfolie (2), einer Kathodenfolie (3) und eines Separators (11) und Tränken mit einer Elektrolytlösung gefertigt wird, mit einem Außengehäuse (10) zur Aufnahme des Kondensatorelements (1) und mit einem Abdichtungsbauteil (9) zum Abdichten eines offenen Teils des Außengehäuses (10), wobei eine Aluminiumtetrafluorid-Salz enthaltende Elektrolytlösung als Elektrolytlösung verwendet wird, und wobei der Separator aus einem wärmebeständigen Kunstharz hergestellt ist,
**dadurch gekennzeichnet, dass** das wärmebeständige Kunstharz Polyester, Polyamid, Vinylon, Rayon, Aramid, Polyethylenterephthalat, Polyethylennaphtahalat, Polyphenylensulfid, aromatisches Polyester, Polyimid, Polyamido-Imido, Polyetherimid, Polytetrafluorethylen, Polyaminobismaleimid, Poly(ethylen-tetraethylen), Poly(vinylidenfluorid), oder eine beliebige Kombination davon ist.

2. Elektrolytkondensator nach Anspruch 1, wobei eine Elektrodenfolie, die einer Phosphatbehandlung unterzogen wird, als die Anodenelektrodenfolie (2) oder die Kathodenelektrodenfolie (3) verwendet wird.

3. Elektrolytkondensator nach Anspruch 1, wobei ein teilweise vernetzender Peroxid-Butylgummi Peroxid als Vernetzungsmittel zu einem Butylgummipolymer hinzufügt, das ein Isobutylen-, Isopren-Copolymer enthält, und Divinylbenzen als Abdichtungsbauteil (9) verwendet wird.

## Revendications

1. Condensateur électrolytique ayant un élément de condensateur (1) fabriqué en enroulant une feuille anode (2), une feuille cathode (3) et un séparateur (11), et en l'imprégnant avec une solution d'électrolyte, un boîtier extérieur (10) pour abriter l'élément de condensateur (1), et un élément de scellement (9) pour sceller une partie ouverte du boîtier extérieur (10), dans lequel une solution d'électrolyte contenant un sel de tétrafluorure d'aluminium est utilisée à titre de solution d'électrolyte, et dans lequel le séparateur est réalisé d'une résine synthétique résistante à la chaleur,
**caractérisé en ce que** la résine synthétique résistante à la chaleur est du polyester, polyamide, vinylon, rayonne, aramide, polyéthylène téréphtalate, polyéthylène naphthalate, sulfure de polyphénylène, polyester aromatique, polyimide, polyamido-imido, polyétherimide, polytétrafluoroéthylène, polyamino bismaléimide, poly(éthylène-tétra éthylène), poly(fluorure de vinylidène), ou une quelconque combinaison de ceux-ci.

2. Condensateur électrolytique selon la revendication 1, dans lequel une feuille électrode soumise à un traitement au phosphate est utilisée à titre de feuille électrode anode (2) ou de feuille électrode cathode (3).

3. Condensateur électrolytique selon la revendication 1, dans lequel un caoutchouc au butyle peroxyde à réticulation partielle qui ajoute peroxyde à titre d'agent de réticulation pour former un polymère de caoutchouc au butyle comprenant un copolymère d'isobutylène, d'isoprène, et du divinylbenzène est utilisé à titre d'élément de scellement (9).
